(19) Europäisches Patentamt
European Patent Office
Office européen des brevets

(11) EP 3 056 815 A1

(12) EUROPEAN PATENT APPLICATION

(43) Date of publication:
**17.08.2016 Bulletin 2016/33**

(51) Int Cl.:
***F23Q 2/16*** *(2006.01)* ***C12P 3/00*** *(2006.01)*

(21) Application number: **16154944.9**

(22) Date of filing: **09.02.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **10.02.2015 IT UB20150244**

(71) Applicant: **Universita' Degli Studi Del Molise**
**86100 Campobasso (IT)**

(72) Inventors:
- **ALFANO, Gabriele**
  **86100 Campobasso (IT)**
- **LUSTRATO, Giuseppe**
  **86100 Campobasso (IT)**
- **RANALLI, Giancarlo**
  **65123 Pescara (IT)**

(74) Representative: **Perrotta, Aldo**
**Ing. Aldo Perrotta**
**Corso Umberto 81**
**88068 Soverato (IT)**

# (54) ORGANIC HYDROGEN GAS LIGHTER

(57) The present invention is relative to the creation of a cigarette lighter that functions using organic hydrogen from the processes of fermenting microbes, using renewable fermentable biomasses such as agricultural residuals or wastewater.

The biological ways of producing hydrogen are represented by the fermentation of organic substances in the presence (photo-fermentation) or in the absence of light (dark-fermentation) .

Organic hydrogen gas lighter of the present invention comprises a container having a refill valve, a release valve of the gas to be turned on and a button that controls the release valve, and ignition means suitable to trigger the combustion of hydrogen with oxygen 'outside air, and is characterized in that the container has a partition wall permeable to only gas which divides said container into two sections, a first section containing a support on which adheres a biomass consisting of fermenting liquids mixed in microorganisms such as heterotrophic bacteria or anaerobic non-photosynthetic bacteria/photosynthetic algae, that in said first section the refill valve is positioned and a second section that contains the hydrogen gas produced by said microorganisms and the release valve of the gas that is opened by the manual device.

EP 3 056 815 A1

## Description

### Technical field of the invention

The present invention is relative to the creation of a biological cigarette lighter that functions using organic hydrogen from the processes of fermenting microbes, using renewable fermentable biomasses such as agricultural residuals or wastewater (aqueous solutions containing sugars, beetroot melasses, honey, fruit juices, vegetable waste liquids and/or shakes, whey, etc.).

### State of the Art

At the beginning of the third millennium approximately 80% of the energy used on the planet comes from fossil fuels. This dependency on fossil energy sources to such a high degree is worrying, for a number of reasons. Above all, we are dealing with sources that are not calculably renewable within a time-scale of several hundreds of years. Secondly, the combustion of such materials causes the freeing into the atmosphere of polluting gasses and particles such as sulfurous anhydride ($SO_2$), nitrogen oxides (NOx), carbon dioxide ($CO_2$) and dust particles.

In the last decades, various laws aimed at limiting emissions have been passed in the industrialized countries. Atmospheric pollution continues to cause concern, however, above all because of the great quantity of carbon dioxide that is emitted into the air and which contributes significantly to the creation of the greenhouse effect with the consequent increase in the average temperature of the earth's surface. The climate change that follows has been the subject of great attention from the international community, so much so that in 1992 the United Nations Organization instituted the Convention on Climate Change, to which numerous countries of the world have adhered.

The reduction of the surface area and volume of the perennial glaciers of the high mountains, together with the melting of those of the two Arctic and Antarctic polar caps and the consequent rise in the sea and ocean levels, are already tangible phenomena; other worrying phenomena are the greater frequency of calamitous events related to torrential and semi-tropical rains, recorded also in areas that in the past had been untouched by them. In the light of as much as has been briefly mentioned, it must be said that a part of the international community does not contest its existence, but on the other hand there is no agreement as to the velocity with which the phenomenon will proceed and thus to the measures to activate.

In this context, hydrogen ($H_2$) appears ever more as a source of energy of great interest; in fact, as a "clean gas", its combustion does not produce dust particles or other gasses, nor carbon dioxide, but only water, a natural component of the biosphere, into which it disperses without causing pollution or any other damage.

For the moment, the energy produced by hydrogen is still very little; the percentage of the total energy produced is, in fact, 3-5%, although a notable increase is foreseen for the future (Das e Veziroglu, 2001; De Philippis 2011).

Currently, hydrogen can be obtained by three diverse methods from three sources; (1) from fossil fuels, (2) from biomasses, (3) from water. Of the 3% produced for industrial uses, 90% is obtained from natural gas and steam reforming and petroleum topping. The gasification of coal and the electrolysis of water represent the other methods, which are, however, less frequently used (Levin et al., 2004).

All these methods however, whether thermochemical or electrochemical, make use of fossil fuel as an energy source to bring about the process and also often as a primary material and are, for the most part, pollutants. Without taking into account that the cost of production is decidedly high.

In GB1401651A a cigarette lighter is described, whose fuel is hydrogen, which is produced elsewhere, probably with the consumption of fossil fuel.

US 3761221A descrive an Hdrogen gas lighter in which hydrogen is produced by electrolysis of electrolyte an therefore it needs of an external power source.

For this reason, great interest is directed towards the production of hydrogen biologically, from biodegradable wastewaters and from agricultural biomass wastes.

Hydrogen is produced spontaneously in nature by Photosynthetic Bacteria (PB, cyanbacteria) and by algae/Fermented Heterotrophic Bacteria (AFHB). The scarce quantities found in nature must be put in relation to the fact that, as the hydrogen is produced by the various fermenting groups, it is immediately used by the microbial populations which live in symbiosis the producers of hydrogen and which, thanks to the high energy content of this gas, use it as an energy source in their own metabolism. This takes place in the soil, in the sediments of the seas, lakes and ponds, in the rumen in which the production of methane takes place largely by the reduction of $CO_2$ caused by $H_2$. The microbial syntrophy in the rumen is so strong that the presence of $H_2$ in the exhalations is considered an indication of a disturbance of the digestive system.

In nature over 50 species of heterotrophic bacterial producers of hydrogen have been identified, but they represent only a minimal part of those which really exist, based on the data reported by biomolecular research, which shows a wide diffusion of the key enzyme in the production of hydrogen, hydrogenase.

The biological way to produce hydrogen does not bring about the consumption of energy, if not in minimal quantities; furthermore it foresees the use, as a primary material, of at least one source of renewable energy, permitting the transformation of waste into resources and thus contributing greatly to limiting the pollution of the environment. The fermenting substrata that can be used are many and of diverse nature and origin; wastewaters, residuals and waste byproducts from the biomasses produced by the agricultural, agro-industrial, food transformation etc., sectors.

The biological ways of producing hydrogen are represented by the fermentation of organic substances in the presence (photo-fermentation) (A) or in the absence of light (dark-fermentation) (B).

A. **Photo-fermentation -** The biological ways of producing hydrogen of type A, in the presence of light, preferably adopt the use of algae and cyanobacteria in which the photosynthetic process, typical of vegetable substances, is adapted for the production of hydrogen. In photosynthesis the absorption of light is due to two distinct photosynthetic processes which, acting together, bring about the decomposition of the water with the development of oxygen (photolysis), coupled with the reduction of carbon dioxide at the expense of the hydrogen.

One of the principle obstacles to the photo-production of hydrogen is the disabling of the system that produces hydrogen by the photo-generated oxygen. Therefore, to make $H_2$ available, the *hydrogenase* enzyme present in both eukaryotic micro-algae (green algae) and prokaryotic (cyanobacteria or blue-green algae) intervenes. The cyanobacteria are also capable of fixing the nitrogen through the nitrogenase enzyme. The result is that in nature there are two metabolic ways to form hydrogen bio-chemically, in the presence of light (A1, A2):

**A1. With hetrocystic bacteria which fix the nitrogen**

$H_2O$→ photo-system→ $(CH_2O)_2$ → ferredoxin→ nitrogenase
↓ ↑ ↓ NADPH ↓
$O_2$ $CO_2$ $CO_2$ $H_2$

**A2. With non-heterocystic bacteria that fix the nitrogen**

$H_2O$→foto-system→ $(CH_2O)_2$ →ferredoxin→ Nitrogenase
↓ ↑ ↓ NADPH or hydrogenase
$O_2$ $CO_2$ $CO_2$ ↑ ↓
Foto-system→ ATP $H_2$

**A3. With phototrophic bacteria/algae.**

Another biological way to produce type A hydrogen, in the presence of light, is adopting, preferably, phototrophic bacteria, which in the literature are rightly considered as the most promising microbial system for the biological production of hydrogen. In fact, such photo-synthetic bacteria and algae have the advantage of a (theoretical) high yield of conversion, of the use of organic material coming from wastewater or refuse, do not produce oxygen and therefore do not have the problems of disabling by oxygen of the other systems, and are able to use a wide spectrum of light.

A general outline for these photo-synthetic processes could be:

$(CH_2O)_2$ → ferredoxin → Nitrogenase→ $H_2$
↑ ATP ↑ ATP

B. **Fermentation in the dark -** Biological methods for the production of type B Hydrogen, in the absence of light (dark fermentation), adopt non-photosynthetic anaerobic heterotrophic bacteria which make use of fermentation processes of organic composts. This production of hydrogen presents numerous advantages from the point of view of a possible industrial production; in fact, the bacteria that induce the fermentation have high yields of hydrogen production from organic substrates, which result as continuous (day and night), independent of light; the microbial kinetics can be opportunely modulated in order to better respond to the necessities of production by making use of, among other things, efficient sources of electrons and the activation of specific hydrogenases.

Other factors that can favourably influence the production yields of bio$H_2$ significantly are the primary raw materials, suitable pre-treatments of the same, fermentation times, the efficiency of microbial consortiums, the temperature of the process, etc.

In bacterial fermentation, the development of bio-hydrogen takes place through the reaction:

**B1.**

$$HCOOH = H_2 + CO_2$$

or

**B2.**

$$NADH + H^+ = H_2 + NAD^+$$

This NADH is produced by the glycolysis of the pyruvate glucose, which in the presence of $CO_2$ generates succinate and formate which consume NADH, with a balance of the residual NADH that can be reassumed as:

NADH = acetate + butyrate + butanediol + acetone succinate – formate

For example, Clostrydium Butirycum can use carbohydrates to produce hydrogen through the formation of organic acids. The anaerobic bacteria are not able to demolish the sugars with hydrogen and $CO_2$ but decompose the carbohydrates with the formation of organic acids that are not further decomposable with hydrogen.

As described above, both the process of photo-fermentation (in the presence of light) and the process of fermentation in the dark (called *dark fermentation*) are becoming the object of ever greater attention by numerous groups of international researchers regarding the possibility of obtaining interesting quantities of bio$H_2$ other than in conditions of light (therefore only in the presence of the sun) but also in the dark (therefore at night, for 24 hours continuously), making the processes interesting and advantageous.

**Description of an embodiment of the invention**

In line with the subject of the present invention, it is represented by the creation and production of a lighter that functions with organic hydrogen.

The new discovery is re-chargeable, therefore functional over time, fueled by a gaseous product that is bio-$H_2$ derived from the fermentation in light or in the dark of wastes and residual fermentable liquids that are therefore suitable. Furthermore, the new discovery will function through the lighting which will take place by the contact of such gas with the sparks generated, in an exemplifying and not limiting example, by the rubbing of a nudded wheel against a flint with the shape of a small cylinder situated inside a charger.

**Equipment of the $H_2$ bio-lighter - Components and materials**

Container: of diverse form, nature and capacity where, for container, a recipient is intended with a limited internal volume, useful and separated from the external ambient (closed), destined to contain: the chosen materials (waste products and liquid residuals) suitable for the production of Bio (Organic)-$H_2$. The container can be created in a transparent material, for example transparent plastic or others, if phototrophic bacteria are used; non-transparent material, non-transparent plastic, ceramics or other materials if heterotrophic bacteria are used. The container is composed of:

- a separating partition of suitable material (a fine membrane, Gore-Tex type fabric, other), that permits the separation into two sections, a first section containing a support to which a biomass constituted of fermenting putrescible liquids mixed with non-photosynthetic heterotrophic anaerobic bacteria or photosynthetic bacteria/algae, a second section that contains gaseous hydrogen produced by said bacteria;
- A separating partition that allows only the passage of the hydrogen produced by the first section to the second section; said partition results therefore capable of separating the liquids and, at the same time, allows the passage of the newly formed bio-hydrogen, that is a gaseous product that creates an overpressure and is able to induce the

passage of the same hydrogen into the second section of the container, where it procedes to establish the same pressure (gas in balance).

- a refill valve in the first section;
- a support for the adhesion-anchorage of the microbial biomass (algae-bacterial) inside the bio-lighter. The support is situated inside the first section destined to hold the fermentable substrate and will function as a fixed anchorage base for the active microbial biomass. The support must present characteristics of great lightness, high porosity and be able to offer high surface areas by volume, must be of inert material, non-bio-degradable, elastic and easily mouldable and adaptable to the internal volume of the sub-tank. Among useful supports, those with a honeycomb structure (for example RECTICEL sponge), offer notable reliability.

The first section of the container will be destined to hold:

The fermentable substrate of diverse origin and nature (wastes and residuals from the food industry, organic vegetable residuals from GDO, agricultural a zoo-technical residuals and wastes), rich in sources of carbon (sugars, fatty acids, etc.), in azote (peptides, amino-acids, etc.) micro and macro nutrients (P, K, Ca, Mg, etc).

- live microbial cultures, for example bacteriaal cultures of E.Coli, Citrobacter spp., Clostridium spp., or others, in the lighter that functions without light; algal/bacterial cultures with live cells of Rhodobacter spp., Synechocystis spp., or others, in the lighter which functions in the light. The cellular suspension (inoculum) is prepared according to the normal microbiological techniques known in the field, that is after the development of the microorganisms chosen in a specific substrate of culture until reaching the desired active cellular biomass.

The initial filling (first charge and successive charges) of the first section of the container of the lighter is with liquids and agri-food wastes rich in fermentable carbohydrates (watery solutions of saccharin, glucose, melasses, whey, etc.). The refill can be facilitated by the use of a suitable refiller (a plastic spray-canister) for small quantities (2-10 ml) and by the use of the recharging device/valve of the standard lighter. The useful quantities that can be inserted are according to the total useful capacity/volume of the bio-lighter.

Once the filling of the lighter's container with liquids and agri-food wastes rich in fermentable carbohydrates has been completed, the biological lighter that functions using hydrogen is ready for use; it will be sufficient to place the device subject of the present invention in the light or in the dark.

In fact, following the microbial fermentation, the organic hydrogen produced will pass from the first section of the container into the second section, both contiguous but divided by a separating partition that is permeable only by the gasses that it will allow to pass.

This will happen because in the first section an over-pressure will be created by the gas that will force the newly-formed organic hydrogen to pass through the semi-permeable separating partition into the second section.

In order to verify the correct functioning of the bio-lighter, at suitable intervals of time and temperature (wide mesophilic temperatures, preferably between 15-37° C), it will be possible to proceed by the usual manual movement of the lighting trigger, by acting on the lighting device, normally placed on the top part of the lighter.

Furthermore, respecting suitable intervals of time between triggering the first flame and successive triggerings will offer the user the possibility of not exceeding in the consumption of tobacco (less use of cigarettes, cigars) while guaranteeing gestures that are often of a psychological and physiological nature towards smoking!

## Claims

1. Organic hydrogen gas lighter comprising a container having a refill valve, a release valve of the gas to be turned on and a button that controls the release valve, and ignition means suitable to trigger the combustion of hydrogen with oxygen 'outside air, **characterized in that** the container has a partition wall permeable to only gas which divides said container into two sections, a first section containing a support on which adheres a biomass consisting of fermenting liquids mixed in microorganisms such as heterotrophic bacteria or anaerobic non-photosynthetic bacteria/photosynthetic algae, that in said first section the refill valve is positioned and a second section that contains the hydrogen gas produced by said microorganisms and the release valve of the gas that is opened by the manual device.

2. Organic hydrogen gas lighter according to claim 1 **characterized in that** the support on which adheres said biomass is inert and non-biodegradable.

3. Organic hydrogen gas lighter according to claim 2 **characterized in that** said support is of inorganic nature, not biodegradable, with cross-linked structure.

4. Organic hydrogen gas lighter according to claim 2 **characterized in that** the container is made of transparent material and that phototropic microorganisms are used.

5. Organic hydrogen gas lighter according to claim 2 **characterized in that** the container is made of non-transparent material and that heterotrophic bacteria are used.

Europäisches Patentamt
European Patent Office
Office européen des brevets

# EUROPEAN SEARCH REPORT

Application Number
EP 16 15 4944

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 3 761 221 A (STILLIONS F) 25 September 1973 (1973-09-25) * column 1, line 65 - column 4, line 4; figure * ----- | 1 | INV. F23Q2/16 C12P3/00 |
| A | US 2007/264534 A1 (ZHANG YI-HENG PERCIVAL [US] ET AL) 15 November 2007 (2007-11-15) * paragraph [0002] - paragraph [0017] * ----- | 1 | |
| A | GB 404 479 A (OTTO REICH; JULIUS VIGNATI) 18 January 1934 (1934-01-18) * page 1, line 90 - page 2, line 72; figures 1-3 * ----- | 1 | |
| A | GB 476 085 A (LUCIEN FREDERIC HENRI PINGEOT) 1 December 1937 (1937-12-01) * page 1, line 96 - page 2, line 78; figures 1-3 * ----- | 1 | |

TECHNICAL FIELDS SEARCHED (IPC)

F23Q
C12P

The present search report has been drawn up for all claims

2

| Place of search | Date of completion of the search | Examiner |
|---|---|---|
| Munich | 15 June 2016 | Theis, Gilbert |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 16 15 4944

This annex lists the patent family members relating to the patent documents cited in the above-mentioned European search report.
The members are as contained in the European Patent Office EDP file on
The European Patent Office is in no way liable for these particulars which are merely given for the purpose of information.

15-06-2016

| Patent document cited in search report | Publication date | Patent family member(s) | Publication date |
|---|---|---|---|
| US 3761221 A | 25-09-1973 | NONE | |
| US 2007264534 A1 | 15-11-2007 | AU 2007249258 A1 | 22-11-2007 |
| | | BR PI0711372 A2 | 01-11-2011 |
| | | CA 2654491 A1 | 22-11-2007 |
| | | EP 2018394 A2 | 28-01-2009 |
| | | JP 2009536829 A | 22-10-2009 |
| | | US 2007264534 A1 | 15-11-2007 |
| | | WO 2007134268 A2 | 22-11-2007 |
| GB 404479 A | 18-01-1934 | NONE | |
| GB 476085 A | 01-12-1937 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- GB 1401651 A
- US 3761221 A